# EUROPEAN PATENT APPLICATION

(11) **EP 4 725 458 A2**
(43) Date of publication of application: **15.04.2026**
(21) Application number: 26161205.5
(22) Date of filing: 19.08.2022
(51) Int. Cl.: A61F 2/72

(54) **TASTE PRESENTATION DEVICE AND TASTE PRESENTATION METHOD**

(30) Priority: 20.08.2021 JP 2021134526
(62) Divisional of application: 22858540.2
(71) Applicant: KIRIN HOLDINGS KABUSHIKI KAISHA, Nakano-ku, Tokyo 164-0001 (JP); Meiji University, Tokyo 101-8301 (JP)
(72) Inventor: SATO, Ai, Tokyo, 164-0001 (JP); MIYASHITA, Homei, Tokyo, 164-8525 (JP)
(74) Representative: Hoffmann Eitle

(57) **Abstract**

In a taste presentation device having a first electrode (11) and a second electrode (12) that are provided to allow an electric circuit to be formed between a food or drink to be taken by a user and a body of the user, and an electric stimulus generation unit (13) that supplies a current for generating an electric stimulation between the first electrode (11) and the second electrode (12), the electric stimulus generation unit (13) includes a noise reduction unit (17A, 17B) that reduces a noise component in the current.

## Description

### TECHNICAL FIELD

The present invention relates to a taste presentation device etc. for producing a change in a taste sensed by a user by means of electric stimulation.

### BACKGROUND ART

Techniques of presenting a taste of a food or drink after altering it to suit a user's preference etc. by means of electric stimulation have been proposed as techniques for enhancing saltiness etc., thereby increasing a feeling of satisfaction for a user who is compelled to follow a bland taste with limited amounts of salt and sugar for the sake of healthcare etc. For example, a taste presentation device has been proposed that produces an enhancing effect on a user's sense of taste by supplying a rectangular wave current of a constant frequency between an anode in contact with a body part of the user, such as the back of the neck, and a cathode in contact with a food or drink (see Patent Literature 1). Another device has been proposed that electrically stimulates a nerve branch that carries a taste sensation to the brain by supplying a two-phase current to an electrode disposed in contact with the tongue etc. of the user (see Patent Literature 2).

### CITATION LIST

### Patent Literature

Patent Literature 1: JP2018-42991A
Patent Literature 2: Japanese Translation of PCT International Application Publication No. 2010-517675

### SUMMARY OF INVENTION

### Technical Problem

An attempt at alteration, such as enhancing saltiness, by means of electric stimulation sometimes leads to a user feeling some kind of unpleasant sensation, like the user feeling an excessive irritating feeling or an unnatural metallic taste. When a current value is lowered to avoid such an inconvenience, with that a saltiness enhancing effect may also be impaired. Thus, there is a possibility that a sufficient alteration effect may fail to be achieved.

Thus, the present invention aims to provide a taste presentation device etc. that can enhance a taste alteration effect while mitigating unpleasant sensations of a user.

### Solution to Problem

A taste presentation device according to one aspect of the present invention is a device which comprises a first electrode and a second electrode that are provided to allow an electric circuit to be formed between a food or drink to be taken by a user and a body of the user, and an electric stimulus generation unit that supplies a current for generating an electric stimulation between the first electrode and the second electrode, wherein the electric stimulus generation unit includes a noise reduction unit that reduces a noise component in the current.

A taste presentation method according to one aspect of the present invention is a method which includes a step of providing a first electrode and a second electrode to allow an electric circuit to be formed between a food or drink to be taken by a user and a body of the user, and a step of supplying a current for generating an electric stimulation between the first electrode and the second electrode, wherein, in the step of supplying the current, a noise component in the current is reduced.

### BRIEF DESCRIPTION OF DRAWINGS

FIG. 1 is a view showing one example of a taste presentation device according to one embodiment of the present invention.
FIG. 2 is a view showing another example of the taste presentation device.
FIG. 3 is a diagram showing an example of the configuration of an electric stimulus generation part.
FIG. 4 is a graph showing one example of a waveform of a current supplied from the electric stimulus generation part.
FIG. 5 is a graph showing another example of the waveform of the current supplied from the electric stimulus generation part.

### DESCRIPTION OF EMBODIMENTS

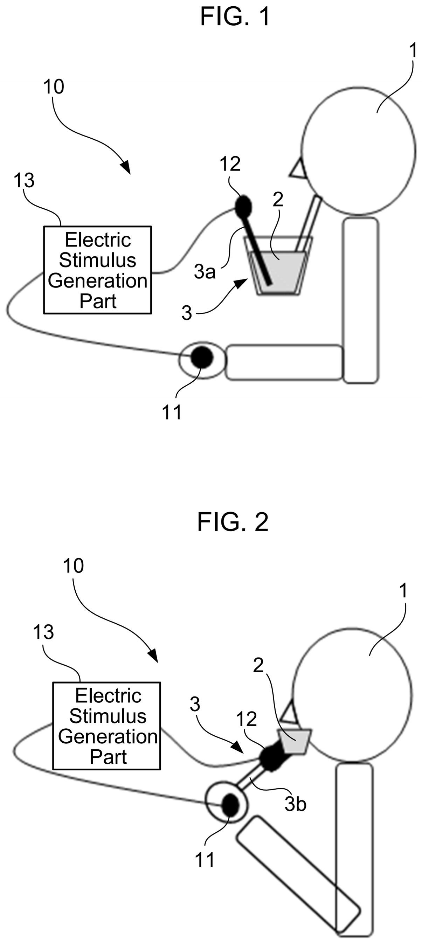

FIG. 1 shows one example of a taste presentation device according to one embodiment of the present invention. A taste presentation device 10 of FIG. 1 includes a first electrode 11 provided in contact with a user 1, a second electrode 12 provided in contact with a food or drink 2 to be taken by the user 1, and an electric stimulus generation part 13 as one example of an electric stimulus unit that supplies a current for generating an electric stimulation between the electrodes 11 and 12.

As one example, the first electrode 11 is put on a hand or an arm of the user 1. A gel-like electrode may be used as the first electrode 11 and this electrode may be attached to the user, or the first electrode 11 may be provided to be held in the user's hand. As one example, the second electrode 12 is put on a piece of conductive tableware 3 that the user uses to eat or drink. The tableware 3 itself may be used as the second electrode 12. For example, as shown in FIG. 1, the second electrode 12 may be put on a muddler 3a immersed in a drink as one example of the food or drink 2, or the muddler 3a itself may be used as the second electrode 12. Alternatively, as shown in FIG. 2, the second electrode 12 may be put on a tool such as a fork, a spoon, or chopsticks (including so-called cutlery) 3b that is used to take a food as one example of the food or drink 2, or these tools 3b may be used as the second electrode 12. In any case, the first electrode 11 and the second electrode 12 may be configured in appropriate forms, as long as these electrodes are provided such that an electric circuit is formed between the food or drink 2 to be taken by the user 1 and the body of the user 1, and thus a current for giving an electric stimulation flows to an organ of the user 1, such as the tongue that senses tastes. The tableware 3 on which the second electrode 12 should be put is not limited to the examples of FIG. 1 and FIG. 2, and dishes, bowls, rice bowls, large bowls, serving bowls, cups, glasses, mugs, goblets, sake cups, deep dishes, jugs, teacups, tumblers, water bottles, chopsticks, forks, spoons, knives, teaspoons and the like, skewers, toothpicks, straws, etc. may be targets to put the second electrode 12 on.

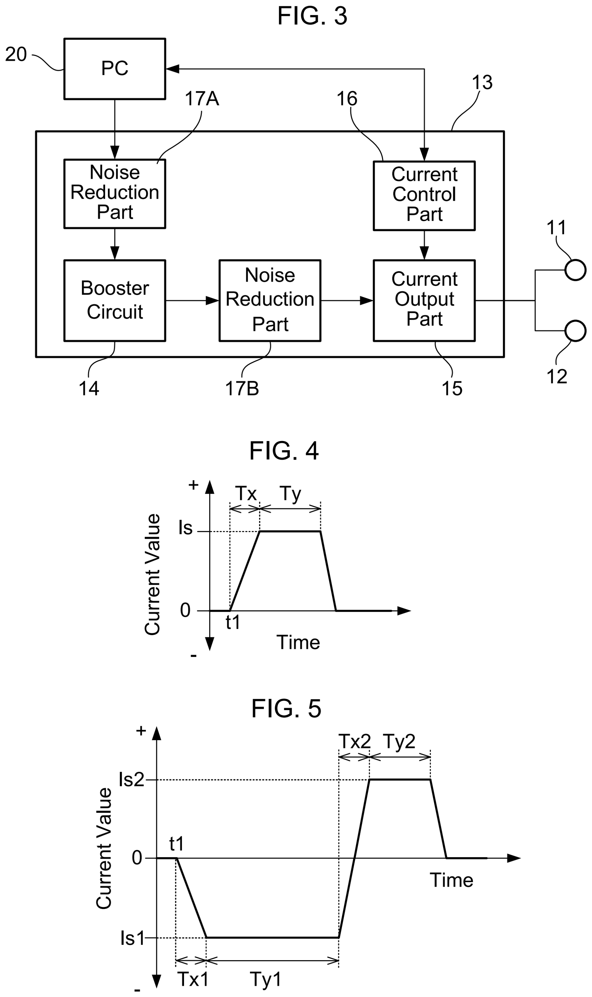

As shown in FIG. 3, the electric stimulus generation part 13 includes a booster circuit 14, a current output part 15, a current control part 16, and noise reduction parts 17A and 17B (which may be represented by reference sign 17). The booster circuit 14 supplies to the current output part 15 a direct current that is supplied, for example, from an external power source (not shown) via a PC (which stands for personal computer) 20 after raising a voltage thereof to a predetermined voltage. As one example, the external power source is an alternating-current commercial power source. The current from the power source is not limited to the example of being supplied via the PC 20, and may instead be supplied through an appropriate converting device having an AC-DC conversion function, such as a converter or an adapter. A direct-current power source, such as a power source battery, may be used as the external power source.

The current output part 15 supplies the current to the electrodes 11 and 12 after shaping the waveform thereof according to a command value given from the current control part 16. The current control part 16 is configured, for example, as a computer unit using a microprocessor, and controls the current supplied to the electrodes 11 and 12 by generating a command value for the current waveform according to setting information on the current waveform given from the PC 20, and outputting this command value to the current output part 15. For example, the current control part 16 can control a direction of the current to allow selection between an anodal stimulation in which the second electrode 12 serves as an anode and a cathodal stimulation in which the second electrode 12 serves as a cathode, as well as can control a magnitude of the current (current value) and a rate of change thereof to target values given from the PC 20. One example of the current control will be described later.

The noise reduction part 17 reduces a noise component included in the current supplied between the electrodes 11 and 12. The noise reduction part 17 is one example of a noise reduction unit. Studies by the present inventors have revealed that a noise component included in a current causes deterioration of an irritating feeling or a metallic taste, with these unpleasant sensations tending to intensify as the noise component becomes larger. Therefore, the noise reduction part 17 is provided to mitigate an influence that a minute noise component has on the irritating feeling or the metallic taste, thereby enhancing a taste alteration effect. Incidentally, the term "reduction" means relatively reducing the noise component in the current compared with when the noise reduction part 17 is not provided, and is a concept that is not limited to a case where the noise component remains to some extent but also encompasses processing such as "removal", "erasure", or "elimination" that reduces the noise component to an undetectable level. "Alteration" is a concept that means changing a taste sensed by the user 1 from an original taste that is felt when an electric stimulation is not applied, and "presentation" is a concept that means making the user 1 sense an altered taste.

The noise reduction part 17 may be configured using various elements used for reducing a noise component in various electric circuits, such as a capacitor, an inductor, or a ferrite core, alone or in appropriate combinations. In the example of FIG. 3, the noise reduction part 17A is provided on an input side of the booster circuit 14 for the purpose of reducing a noise component originating from the external power source, and the noise reduction part 17B is provided on an output side of the booster circuit 14 for the purpose of removing a noise component corresponding to an internal oscillation frequency that is generated when the booster circuit 14 raises the voltage. However, the positions and the number of the noise reduction parts 17 may be changed as appropriate according to an internal configuration of the electric stimulus generation part 13, such as the position of a noise generation source.

Next, examples of the current control by the current control part 16 will be described. FIG. 4 shows one example of the current waveform controlled by the current control part 16, with the axis of abscissa and the axis of ordinate representing time and a current value, respectively. The current value is indicated as a positive value when the second electrode 12 serves as the anode, and as a negative value when the second electrode 12 serves as the cathode. In the following description, a change in the current value in a direction in which the absolute value becomes larger will be expressed as an "increase", regardless of whether the change is in a positive or negative direction.

In the example of FIG. 4, it is assumed that an electric circuit including the user 1 is formed at time t1 as the user 1 takes the food or drink 2 and the electrodes 11 and 12 electrically conduct with each other. When the formation of the electric circuit is detected, the current control part 16 starts to supply the current with the direction of the current set for the anodal stimulation, i.e., such that the first electrode 11 serves as the cathode while the second electrode serves as the anode, and gradually increases the current value to a set value Is over a predetermined increase time Tx. After the set value Is is reached, the current control part 16 holds the set value Is for a predetermined holding time Ty, and when the holding time Ty has elapsed, stops the current supply. A rate of increase (a rate of change) in the current value during the increase time Tx is restricted to a low rate compared with a rate of increase of when the current value is immediately increased to the set value Is, in other words, a rate of increase of when a speed of increase in the current value is not restricted and the current value is increased with a maximum speed that is determined by electric characteristics of the electric stimulus generation part 13. Thus, increasing the current value moderately slowly can weaken an irritating feeling sensed by the user 1.

The set value Is of the current value and the rate of increase in the current value until reaching the set value Is may be set as appropriate according to an environmental context, such as individual differences of the user 1, an intended degree of the taste alteration effect, or the type of the target food or drink 2 to be taken. As one example, the absolute value of the set value Is can be set to the degree such as 0.1 mA to 1.0 mA. From the viewpoint of enhancing the taste alteration effect, the absolute value of the set value Is is preferably 0.1 mA to 1.0 mA, more preferably 0.3 mA to 1.0 mA, further preferably 0.5 mA to 1.0 mA, and even more preferably 0.7 mA to 1.0 mA. The rate of increase can be set to a range of 0.1 mA/ms or lower. When the set value Is is set to the aforementioned degree, the increase time Tx may be set to at least 0.01 seconds or longer as a guide, or may be set to 0.1 seconds or longer or 0.3 seconds or longer depending on circumstances. The holding time Ty of the set value Is may also be set as appropriate according to the environmental context, such as the individual differences of the user 1, the intended degree of the taste alteration effect, or the type of the target food or drink 2 to be taken. As one example, the holding time Ty may be set to about 0.5 seconds.

FIG. 5 shows another example of the current waveform controlled by the current control part 16. The axis of abscissa and the axis of ordinate are the same as in the case of FIG. 4, and that an electric circuit including the user 1 is formed at time t1 is also the same as in FIG. 4. The example of FIG. 5 differs from the example of FIG. 4 in that both the cathodal stimulation and the anodal stimulation are implemented by reversing the polarity of the electrodes 11 and 12 during the current supply. Specifically, in the example of FIG. 5, when the formation of the electric circuit is detected at time t1, the current control part 16 starts to supply the current with the direction of the current set for the cathodal stimulation, i.e., such that the first electrode 11 serves as the anode while the second electrode 12 serves as the cathode, and gradually increases the current value to a set value Is1 over a predetermined increase time Tx1. After the set value Is1 is reached, the current control part 16 holds the set value Is1 for a predetermined holding time Tyl. When the holding time Ty1 has elapsed, the current control part 16 changes the direction of the current such that the first electrode 11 serves as the cathode while the second electrode 12 serves as the anode, and increases the current value of the second electrode 12 to a set value Is2 over a predetermined reversal time Tx2. After the set value Is2 is reached, the current control part 16 holds the set value Is2 for a predetermined holding time Ty2, and when the holding time Ty2 has elapsed, stops the current supply.

When the polarity is reversed after the current supply is started as in FIG. 5, it is possible to restrain the current values Is1, Is2 to relatively low values, and yet to sufficiently induce a taste changing action by means of the polarity reversal, thereby enhancing the taste alteration effect. In the cathodal stimulation, the unpleasant sensations of the user 1 can be mitigated to a comparatively low degree while the taste alteration effect is weak, whereas in the anodal stimulation, the taste alteration effect is comparatively high while the unpleasant sensations, especially the metallic taste tends to be induced comparatively intensely. Therefore, by implementing the cathodal stimulation first to mitigate the unpleasant sensations and then implementing the anodal stimulation by reversing the polarity, it is possible to secure a large amount of change in the current value during the reversal to thereby enhance the taste alteration effect, and at the same time to restrain the set value Is2 of the current during the anodal stimulation to a relatively low level to thereby restrain the unpleasant sensations from increasing.

In the example of FIG. 5, various parameters that specify the current waveform, namely the set values Is1 and Is2 of the current value, the rates of increase in the current value until reaching the set values Is1 and Is2, and the holding time Ty1 of the cathodal stimulation may be set as appropriate according to the environmental context, such as the individual differences of the user 1, the intended degree of the taste alteration effect, or the type of the target food or drink 2 to be taken. As one example, the absolute value of each of the set values Is1 and Is2 can be set to about 0.1 mA to 1.0 mA. The absolute values of the set values Is1 and Is2 may be equal to each other or may be different from each other. From the viewpoint of enhancing the taste alteration effect, the absolute values of the set values Is1 and Is2 are preferably 0.1 mA to 1.0 mA, more preferably 0.3 mA to 1.0 mA, further preferably 0.5 mA to 1.0 mA, and even more preferably 0.7 mA to 1.0 mA.

With respect to the rate of increase in the current value until the current value reaches the set value Is1 during the cathodal stimulation, it can be set to a range of 0.1 mA/ms or shorter. When the set value Is1 is set to the aforementioned degree, the increase time Tx1 may be set to at least 0.01 seconds or longer as a guide, and may be set to 0.1 seconds or longer or 0.3 seconds or longer depending on circumstances. As one example, the holding time Ty1 of the set value Is1 may be set to at least 0.1 seconds or longer as a guide, and may be set to 0.3 seconds or longer, or 0.5 seconds or longer, or further 1 second or longer depending on circumstances. When a longer holding time Ty1 is secured, effects of the cathodal stimulation are more likely to be achieved.

The reversal time Tx2 required for the set value to change from Is1 to Is2 may be set to 0.5 seconds or shorter as a guide. There is a possibility that when 0.5 seconds are exceeded, effects of the reversal may fail to be sufficiently achieved. The reversal time Tx2 may be set to preferably 0.4 seconds or shorter. On the other hand, from the viewpoint of reducing the irritating feeling, the reversal time Tx2 may be set to preferably 0.01 seconds or longer and further suitably 0.1 seconds or longer. The holding time Ty2 of the set value Is2 during the anodal stimulation may be set as appropriate according to the environmental context, such as the individual differences of the user 1, the intended degree of the taste alteration effect, or the type of the target food or drink 2 to be taken.

The current waveforms shown in FIG. 4 and FIG. 5 are examples, and the waveform of the current to be supplied between the electrodes 11 and 12 may be modified or changed as appropriate. For example, while only the anodal stimulation is implemented in the example of FIG. 4, only the cathodal stimulation may be implemented. While the cathodal stimulation is implemented first and thereafter the anodal stimulation is implemented by reversing the polarity of the electrodes 11 and 12 in the example of FIG. 5, this order may be switched to implement the anodal stimulation first and thereafter implement the cathodal stimulation. The current waveform may be set with various factors taken into account, such as a purpose of giving an electric stimulation, i.e., a viewpoint of how the taste should be altered before being presented to the user 1, the type of the target food or drink 2 of which the taste is to be altered, and preferences and other individual characteristics of the user 1. The rate of increase in the current value during the increase time Tx in the example of FIG. 4 and the rates of increase in the current value during the increase time Tx1 and the reversal time Tx2 in the example of FIG. 5 are all constant, and the change in the current value is a linear change with a constant inclination, but the change in the current value may be a change having a quadratic curve shape or another nonlinear shape. Also, during the holding times Ty, Ty1, and Ty2, the current value may be changed within an allowable range instead of being fixed at a constant value. For example, the current waveform may be set to an appropriate waveform such as a sinusoidal waveform or a saw-tooth form. Also in such cases, it is possible to achieve a reducing effect on the unpleasant sensations by providing the noise reduction unit, as well as to reduce the irritating feeling by increasing the current value comparatively gradually. Further, when a current waveform involving polarity reversal is set, it is possible to restrain the current value to a relatively low level and yet to increase the amount of change in the current value and thereby enhance the taste alteration effect, regardless of which of the cathodal stimulation and the anodal stimulation is implemented first.

Target foods and drinks of which the tastes are to be presented by the present invention may include various foods and drinks, as long as the current for generating the electric stimulation can be passed therethrough. While any foods and drinks that contain moisture can carry the current, foods and drinks that contain preferably 20 weight% or more, more preferably 25 weight% or more, and further preferably 30 weight% or more moisture can be used as targets. An altering effect on tastes can be exerted on various foods and drinks, as long as these foods and drinks contain a taste component. For example, when enhancing the saltiness of a food or drink containing salt, the present invention can be suitably applied to foods and drinks that contain 0.05 weight% or more salt, and the present invention can be applied to foods and drinks that contain more preferably 0.1 weight% or more and further preferably 0.15 weight% or more salt. As for specific foods and drinks, the following can be named as targets of which the tastes are to be altered by the present invention: noodles, such as ramen; soups; meat dishes and processed meat products, such as sausages and fried meat; fish dishes, such as grilled fish and fish eggs, and processed seafood products; egg dishes and processed egg products; tofu dishes and processed tofu products; cheeses; cooked products; vegetable dishes and processed vegetable products; flour dishes; fried foods; one-pot dishes; stew, such as curry; alcoholic drinks, such as beer and wine; non-alcoholic drinks, such as flavored water drinks, sports drinks (isotonic drinks), fruit juice drinks, milk drinks, vegetable juice drinks, acidic drinks, carbonated drinks, whey drinks, coffee drinks, tea drinks, green tea drinks, oolong tea drinks, barley tea drinks, energy drinks, and beer-taste drinks; and others. The target taste to be altered is not limited to saltiness, and various tastes such as sweetness, acidity, umami, bitterness, astringency, acridity, fattiness, carbonic acidity, and alcoholicity can be presented after being altered by the present invention.

Any trained panelists can easily and definitely decide a degree of a taste (e.g., saltiness) enhancing effect and degrees of intensity of the irritating feeling and the metallic taste in the present invention. Ordinary methods can be used for evaluation criteria and how to sum up evaluations among panelists. While the number of panelists who evaluate the taste (e.g., saltiness) enhancing effect and the degrees of intensity of the irritating feeling and the metallic taste in the present invention may be one, from the viewpoint of obtaining evaluations with higher objectivity, the lower limit of the number of panelists can be set to, for example, two or more and preferably three or more, and from the viewpoint of more simply conducting an evaluation test, the upper limit of the number of panelists can be set to, for example, 20 or less or 10 or less. For evaluations of the taste (e.g., saltiness) enhancing effect and the degrees of intensity of the irritating feeling and the metallic taste when the number of panelists is two or more, averages of evaluations given by all the panelists to the taste (e.g., saltiness) enhancing effect and the degrees of intensity of the irritating feeling and the metallic taste for that food or drink sample may be adopted. When the evaluation criteria are each assigned a score of an integer, average values of scores given by all the panelists may be adopted as the evaluations of the taste (e.g., saltiness) enhancing effect and the degrees of intensity of the irritating feeling and the metallic taste. When the average values of the scores are adopted as just described, values obtained by rounding these average values to the first decimal place or the second decimal place (preferably the second decimal place) may be adopted. Incidentally, when the number of panelists is two or more, to reduce variation in evaluation among the panelists, it is preferable that work of standardizing the evaluation criteria such that the evaluation criteria of the panelists become as uniform as possible be performed before an actual sensory evaluation test is performed. Such standardization work may involve each panelist evaluating the strengths of the tastes of a plurality of types of food and drink samples on which the taste (e.g., saltiness) enhancing effect is known, or evaluating the degrees of intensity of the irritating feeling and the metallic taste, and then comparing the scores to confirm that no significant divergence arises in the evaluation criteria of the panelists. Further, it is preferable that through such prior standardization work relating to the evaluation criteria, standard deviation of the evaluations given by the panelists to the taste (e.g., saltiness) enhancing effect and the degrees of intensity of the irritating feeling and the metallic taste be restricted to 0.5 or less when the scores are given on a scale of multiple levels (e.g., on a scale of five levels of 1, 2, 3, 4, and 5).

### Examples

Next, tests that were conducted to confirm effects of the present invention will be described. Test conditions are as follows.

### (1) Test Conditions

### [Devices Used]

- Device 1: A device in which the electrodes were configured as shown in FIG. 1 and the electric stimulus generation part was configured as shown in FIG. 3. Provided that a 100 V alternating-current commercial power source was used as the external power source, and a current was supplied to the booster circuit after being converted into a 20 V, 2.25 A direct current by an AC adapter.
- Device 2: A device in which, as compared with Device 1, the configuration of the electrodes was replaced with that shown in FIG. 2.
- Device 3: A device in which, as compared with Device 1, the noise reduction parts were omitted.
- Device 4: A device in which, as compared with Device 2, the noise reduction parts were omitted.

In all of Devices 1 to 4, as the first electrode, a product named "Red Dot" (Registered trademark), monitoring electrode 2560 manufactured by 3M Japan Limited was used. The first electrode was attached to a subject's hand. As the second electrode, an electrode that could be put on a target to be connected like, for example, a clip electrode was used, and this electrode was directly connected to a piece of conductive tableware. As for the noise reduction parts, as the noise reduction part 17A for the purpose of reducing a noise component originating from the external power source, an inductor with a part number LQM21PN4R7MGRD manufactured by Murata Manufacturing Co., Ltd. was used, and as the noise reduction part 17B corresponding to the internal oscillation frequency of the booster circuit, an inductor MLZ2012 type, part number ML22012N101LT000 manufactured by TDK Corporation was used.

### [Food and Drink Samples]

As target food and drink samples to be taken, the following were prepared:
- Drink tasting sample: Salt solutions with a salt concentration of 0.3 weight% and 0.5 weight% were prepared by reference to the salt concentrations of soups having normal to bland tastes.
- Food tasting sample: A salt solution-agar gel with a salt concentration of 0.6 weight% (agar concentration: 0.8 weight%) was prepared by reference to the salt concentrations of home-cooked grilled foods having normal to bland tastes, and this gel was cut into uniform 1 cm cubes to use as food tasting samples.
- General food samples: A sports drink, instant egg soup, instant miso soup, fish sausage, steamed chicken, and cup noodle that were commercially available were used. The cup noodle was adjusted to a bland state by diluting the soup twofold.

### [Test Method]

Four subjects who had been confirmed by a prior examination as being able to distinguish a difference in the strength of saltiness equivalent to a salt concentration of 0.03 weight% were selected, and each subject was asked to take the food and drink samples using Devices 1 to 4 as appropriate. In selecting the subjects, the following work was performed to reduce variation in evaluation among the subjects before an actual sensory evaluation test was conducted: For the saltiness enhancing effect and the degrees of intensity of the irritating feeling and the metallic taste, each subject evaluated the strengths of the tastes of a plurality of types of known food and drink samples, or the degrees of the irritating feeling and the metallic taste upon applying to the subject's tongue an electrode that presented a plurality of types of electric stimulation conditions under which the degrees of intensity of the irritating feeling and the metallic taste were known, and then the scores were compared to confirm that no significant divergence arose in the evaluation criteria of each subject. Further, it was also confirmed that standard deviation of evaluations given by the subjects to the saltiness enhancing effect and the degrees of intensity of the irritating feeling and the metallic taste was 0.5 or less. A current for taste stimulation was supplied between the electrodes from the electric stimulus generation part during ingestion, and each subject was asked to give a point to each of the saltiness enhancing effect, the irritating feeling, and the metallic taste according to the criteria of Tables 1 to 3 below to thereby acquire sensory evaluations. Then, values obtained by rounding average values of the scores of all the subjects to the first decimal place were used as evaluation results. Regarding current control, a test was conducted for each of a case where only the anodal stimulation was performed (the example of FIG. 4), a case where only the cathodal stimulation was performed (corresponding to a waveform that is a vertical reversal of FIG. 4), and a case where the cathodal stimulation was performed first and thereafter the polarity was reversed for the anodal stimulation as illustrated in FIG. 5.

The increase time Tx and the holding times Ty and Ty1 shown in FIG. 4 or FIG. 5 were also varied as appropriate to check the influence.

**Table 1**

| Score | Criteria |
|---|---|
| 5 | Increased 40% or more |
| 4 | Increased 30 to 40% |
| 3 | Increased 20 to 30% |
| 2 | Increased 10 to 20% |
| 1 | Increased 1 to 10% |
| 0 | Not changed |

**Table 2**

| Score | Criteria |
|---|---|
| 5 | Unbearable |
| 4 | Bearable in test, but unbearably in daily |
| 3 | May be sensed in daily, but not unbearably |
| 2 | Not enough to be sensed |
| 1 | Do not be sensed |

**Table 3**

| Score | Criteria |
|---|---|
| 5 | Metaric taste is strogly sensed |
| 4 | Metaric taste is sensed, and taste of food or drink is affected |
| 3 | Metaric taste is sensed, but taste of food or drink is not affected |
| 2 | Metaric taste is felt if pointed out, but not enough to be sensed |
| 1 | Do not feel |

The saltiness enhancing effect of Table 1 was evaluated as "effective" when the point was three or higher, and each of the irritating feeling of Table 2 and the metallic taste of Table 3 was evaluated as "no problem" when the point was three or lower. In the test results shown below, cases where the saltiness enhancing effect was evaluated at three or higher points and moreover the irritating feeling and the metallic taste were evaluated at three or lower points will be referred to as Examples, and test results that do not apply will be referred to as Comparisons.

### (2) Confirmation of Effects of Noise Reduction

A test that was conducted to check an influence that noise reduction had on the saltiness enhancing effect etc. will be described with reference to Table 4. Examples 1 to 12 of Table 4 show evaluation results of cases where either Device 1 or Device 2 including the noise reduction parts was used and the current was supplied between the electrodes to generate either the anodal stimulation or the cathodal stimulation. Comparisons 1 to 4 show evaluation results of cases where either Device 3 or Device 4 from which the noise reduction parts were omitted was used and the current was supplied between the electrodes to generate either the anodal stimulation or the cathodal stimulation.

**Table 4**

| | Device Used | Sample | Electric Stimulation Condition | Saltiness | Irritating Feeling | Metaric Taste |
|---|---|---|---|---|---|---|
| Example 1 | Device 1 | 0.5weight% Salt Solution | Anodal Stimulation 0.3mA, Hold 0.5seconds | 3 | 1 | 1 |
| Example 2 | Device 1 | 0.5weight% Salt Solution | Anodal Stimulation 0.5mA, Hold 0.5seconds | 4 | 1 | 2 |
| Example 3 | Device 1 | 0.5weight% Salt Solution | Anodal Stimulation 0.7mA, Hold 0.5seconds | 4 | 1 | 2 |
| Example 4 | Device 1 | 0.5weight% Salt Solution | Anodal Stimulation 1.0mA, Hold 0.5seconds | 4 | 1 | 3 |
| Example 5 | Device 1 | 0.5weight% Salt Solution | Cathodal Stimulation 0.3mA, Hold 0.5seconds | 3 | 1 | 1 |
| Example 6 | Device 1 | 0.5weight% Salt Solution | Cathodal Stimulation 0.5mA, Hold 0.5seconds | 3 | 1 | 1 |
| Example 7 | Device 1 | 0.5weight% Salt Solution | Cathodal Stimulation 1.0mA, Hold 0.5seconds | 3 | 1 | 1 |
| Example 8 | Device 1 | 0.3weight% Salt Solution | Anodal Stimulation 0.5mA, Hold 0.3seconds | 4 | 1 | 2 |
| Example 9 | Device 1 | 0.3weight% Salt Solution | Cathodal Stimulation 0.5mA, Hold 0.5seconds | 3 | 1 | 1 |
| Example 10 | Device 2 | 0.6weight% Salt Solution-Agar Gel | Anodal Stimulation 0.5mA, Hold 0.3seconds | 4 | 2 | 2 |
| Example 11 | Device 2 | 0.6weight% Salt Solution-Agar Gel | Cathodal Stimulation 0.5mA, Hold 0.5seconds | 3 | 2 | 1 |
| Comparison 1 | Device 3 | 0.5weight% Salt Solution | Anodal Stimulation 0.3mA, Hold 0.5seconds | 2 | 3 | 5 |
| Comparison 2 | Device 3 | 0.5weight% Salt Solution | Anodal Stimulation 1.0mA, Hold 0.5seconds | 3 | 5 | 5 |
| Comparison 3 | Device 3 | 0.5weight% Salt Solution | Cathodal Stimulation 1.0mA, Hold 0.5seconds | 2 | 5 | 1 |
| Comparison 4 | Device 4 | 0.6weight% Salt Solution-Agar Gel | Anodal Stimulation 0.5mA, Hold 0.3seconds | 3 | 4 | 4 |

"Electric Stimulation Condition" of Table 4 shows distinction as to which of the anodal stimulation or the cathodal stimulation was implemented, the set value of the current, and the holding time of the current at the set value. For example, "anodal stimulation, 0.3 mA, 0.5s holding" of Example 1 shows that the anodal stimulation shown in FIG. 4 was implemented, with the set value Is being 0.3 mA and the holding time Ty being 0.5 seconds. Examples 1 to 9 are examples in which an electric stimulation was applied to the drink tasting sample using Device 1, and Examples 10 and 11 are examples in which an electric stimulation was applied to the drink tasting sample using Device 2. The increase time Tx of the current value was set to 0.01 seconds in all of Examples 1 to 11. Since the set value Is is set to one of 0.3 mA (Examples 1 and 5), 0.5 mA (Examples 2, 6, and 8 to 11), 0.7 mA (Example 3), and 1.0 mA (Example 4), the rate of increase in the current value during the increase time Tx is within a range of 0.03 mA/ms to 0.1 mA/ms. On the other hand, Comparisons 1 to 4 are cases in which Device 3 or Device 4 not equipped with the noise reduction parts was used. In terms of the food and drink samples and the electric stimulation conditions, Comparison 1 is the same as Example 1; Comparison 2 is the same as Example 4; Comparison 3 is the same as Example 7; and Comparison 4 is the same as Example 10.

As is clear from Table 4, it was confirmed that in Examples 1 to 11 in which noise reduction was implemented, adequate evaluations could be obtained on each of the saltiness enhancing effect, the irritating feeling, and the metallic taste, regardless of the anodal stimulation or the cathodal stimulation. On the other hand, in Comparisons 1 to 4 in which noise reduction was not implemented, the evaluations were inadequate despite the electric stimulation conditions being the same as in corresponding Examples 1, 4, 7, and 10. In particular, a sufficient effect failed to be achieved with respect to the irritating feeling or the metallic taste.

### (3) Confirmation of Effects of Polarity Reversal

A test that was conducted to check changes in the saltiness enhancing effect etc. resulting from reversing the polarity of the electrodes will be described with reference to Table 5. Examples 12 to 22 of Table 5 show evaluation results of cases where Device 1 was used for the drink tasting sample and Device 2 was used for the food tasting sample, respectively, and the cathodal stimulation was implemented first according to the waveform of FIG. 5 and thereafter the anodal stimulation was implemented by reversing the direction of the current.

**Table 5**

| | Device Used | Sample | Electric Stimulation Condition | Saltiness | Irritating Feeling | Metaric Taste |
|---|---|---|---|---|---|---|
| Example 12 | Device 1 | 0.5weight% Salt Solution | Cathodal Stimulation 0.7mA, Hold 1.0seconds, | 5 | 1 | 1 |
| | | | Reveral within 0.1seconds or shorter, Anodal Stimulation 0.7mA, Hold 0.5seconds | | | |
| Example 13 | Device 1 | 0.5weight% Salt Solution | Cathodal Stimulation 1.0mA, 1.0seconds, | 5 | 2 | 2 |
| | | | Revesal within 0.1second or shorter, Anodal Stimulation 1.0mA, Hold 0.5seconds | | | |
| Example 14 | Device 1 | 0.5weight% Salt Solution | Cathodal Stimulation 1.0mA, Hold 0.01seconds, | 4 | 2 | 3 |
| | | | Reversl within 0.1seconds or shorter, Anodal Stimulation 1.0mA, Hold 0.5seconds | | | |
| Example 15 | Device 1 | 0.5weight% Salt Solution | Cathodal Stimulation 1.0mA, Hold 0.1seconds, | 4 | 2 | 2 |
| | | | Reversl within 0.1seconds or shorter, Anodal Stimulation 1.0mA, Hold 0.5seconds | | | |
| Example 16 | Device 1 | 0.5weight% Salt Solution | Cathodal Stimulation 1.0mA, Hold 0.3seconds, | 5 | 2 | 2 |
| | | | Reversl within 0.1seconds or shorter, Anodal Stimulation 1.0mA, Hold 0.5seconds | | | |
| Example 17 | Device 1 | 0.5weight% Salt Solution | Cathodal Stimulation 1.0mA, Hold 0.5seconds, | 5 | 2 | 2 |
| | | | Reversl within 0.1seconds or shorter, Anodal Stimulation 1.0mA, Hold 0.5seconds | | | |
| Example 18 | Device 1 | 0.5weight% Salt Solution | Cathodal Stimulation 1.0mA, 1.0seconds, | 5 | 2 | 1 |
| | | | Reversl within 0.1seconds or shorter, Anodal Stimulation 0.5mA, Hold 0.5seconds | | | |
| Example 19 | Device 1 | 0.5weight% Salt Solution | Cathodal Stimulation 1.0mA, 1.0seconds, | 5 | 2 | 1 |
| | | | Reversl within 0.1seconds or shorter, Anodal Stimulation 0.3mA, Hold 0.5seconds | | | |
| Example 20 | Device 1 | 0.5weight% Salt Solution | Cathodal Stimulation 1.0mA, 1.0seconds, | 4 | 1 | 1 |
| | | | Reversl within 0.1seconds or shorter, Anodal Stimulation 0.1mA, Hold 0.5seconds | | | |
| Example 21 | Device 1 | 0.3weight% Salt Solution | Cathodal Stimulation 0.5mA, Hold 0.5seconds, | 5 | 1 | 1 |
| | | | Reversl within 0.1seconds or shorter, Anodal Stimulation 0.5mA, Hold 0.3seconds | | | |
| Example 22 | Device 2 | 0.6weight% Salt Solution-Agar Gel | Cathodal Stimulation 0.5mA, Hold 0.5seconds, | 5 | 2 | 1 |
| | | | Reversl within 0.1seconds or shorter, Anodal Stimulation 0.5mA, Hold 0.3seconds | | | |

"Electric Stimulation Condition" of Table 5 show settings of the set values Is1 and Is2 and the holding times Ty1 and Ty2 of FIG. 5. For example, it is shown that in Example 12, the set value Is1 of the current value during the cathodal stimulation is -0.7 mA; that the holding time Ty1 thereof is 1.0 seconds; that the set value Is2 of the current value during the anodal stimulation is +0.7 mA; and that the holding time Ty2 thereof is 0.5 seconds. Correspondence relationships between the samples and the anodal stimulation conditions in Examples 12 to 22 and the samples and the anodal stimulation conditions in Examples 1 to 11 of Table 4 are as follows: Example 12 corresponds to Example 3; Examples 13 to 17 correspond to Example 4; Example 18 corresponds to Example 2; Example 19 corresponds to Example 1; Example 21 corresponds to Example 8; and Example 21 corresponds to Example 9. Example 20 is an example in which the holding time Ty2 of the anodal stimulation was set to the same time as in Example 19, while the set value Is2 of the current value was further reduced from 0.3 mA to 0.1 mA. Incidentally, the increase time Tx1 of the current value when starting the cathodal stimulation is 0.01 seconds, and the rate of increase is within the same range as in Examples 1 to 11 of Table 4. The reversal time Tx2 was set to 0.1 seconds or shorter in all of Examples 12 to 22.

When the evaluation results were compared according to the aforementioned correspondence relationships, a further improvement on the evaluation results of Examples shown in Table 4 was confirmed in each case except for Example 14. For example, in Example 3, the evaluations of the saltiness enhancing effect, the irritating feeling, and the metallic taste is "4, 1, 2", whereas in Example 12, the evaluations are "5, 1, 1" and thus the saltiness enhancing effect and the metallic taste are improved. It was also confirmed that when polarity reversal was implemented, the whole flavor of the food and drink sample was enhanced, and that consequently the metallic taste was reduced compared with when the anodal stimulation was implemented alone. Incidentally, as for Example 14, the evaluations are almost equivalent to those of when the anodal stimulation was implemented alone. A possible reason for this is that as the holding time of the cathodal stimulation was as extremely short as 0.01 seconds, effects of implementing the cathodal stimulation first failed to be sufficiently exerted. Therefore, it is desirable that the cathodal stimulation be held for 0.01 seconds or longer as a guide for a minimum. On the other hand, as is clear from Example 20, when the polarity was reversed, the saltiness enhancing effect was enhanced compared with Example 1, despite the set value of the current value during the anodal stimulation being as low as 0.1 mA. Also from this, it can be seen that polarity reversal is effective as means for enhancing the taste alteration effect while restraining the set value of the current value.

### (4) Confirmation of Influence of Type of Food or Drink on Effects

A test that was conducted to check differences in the saltiness enhancing effect etc. according to the type of food or drink will be described with reference to Table 6. Table 6 shows results of evaluating the saltiness enhancing effect etc. with Device 1 to Device 4 selectively used for various general food samples in place of the drink tasting sample or the food tasting sample.

**Table 6**

| | Device Used | Sample | Electric Stimulation Condition | Saltiness | Irritating Feeling | Metaric Taste | Comment |
|---|---|---|---|---|---|---|---|
| Example 23 | Device 1 | Commercial Sports Drink | Anodal Stimulation 1.0mA, Hold 0.5seconds | 4 | 1 | 2 | Sensed saltiness strongly when drinking. |
| Example 24 | Device 1 | Commercial Sports Drink | Cathodal Stimulation 1.0mA, Hold 0.5seconds | 4 | 1 | 1 | Not only the saltiness but also the wholel flavor is enhanced. |
| Example 25 | Device 1 | Commercial Sports Drink | Cathodal Stimulation 0.5mA, Hold 1.0seconds thereafter Anodal Stimulation 0.5mA, Hold 1.0seconds | 5 | 2 | 1 | Not only the saltiness but also the whole flavor is enhanced. |
| Compa rison 5 | Device 3 | Commercial Sports Drink | Anodal Stimulation 1.0mA, Hold 0.5seconds | 3 | 4 | 5 | Metaric tase is strong when drinking. |
| Compa rison 6 | Device 3 | Commercial Sports Drink | Cathodal Stimulation 1.0mA, Hold 0.5seconds | 2 | 4 | 1 | Not only the saltiness but also the whole flavor is slightly enhanced,but irritating feeling is strong when drinking. |
| Example 26 | Device 1 | Commercial Instant Egg Soup | Cathodal Stimulation 0.5mA, Hold 1.0seconds thereafter Anodal Stimulation 0.5mA, Hold 1.0seconds | 5 | 2 | 1 | Not only the saltiness but also the whole flavor is enhanced. |
| Example 27 | Device 1 | Commercial Instant Egg Soup | Cathodal Stimulation 0.5mA, Hold 1.0seconds thereafter Anodal Stimulation 0.3mA, Hold 1.0seconds | 4 | 2 | 1 | Not only the saltiness but also the whole flavor is enhanced. |
| Example 28 | Device 1 | Commercial Instant Miso Soup | Cathodal Stimulation 0.5mA, Hold 1.0seconds thereafter Anodal Stimulation 0.5mA, Hold 1.0seconds | 4 | 2 | 1 | Not only the saltiness but also the whole flavor is enhanced. |
| Example 29 | Device 2 | Commercial Fish Sausage | Anodal Stimulation 1.0mA, Hold 0.5seconds | 4 | 3 | 2 | Sensed strong saltiness. |
| Example 30 | Device 2 | Commercial Fish Sausage | Cathodal Stimulation 1.0mA, Hold 0.5seconds | 3 | 3 | 1 | Not only the saltiness but also the whole flavor is enhanced. |
| Example 31 | Device 2 | Commercial Fish Sausage | Cathodal Stimulation 0.5mA, Hold 1.0seconds thereafter Anodal Stimulation 0.5mA, Hold 1.0seconds | 5 | 2 | 1 | Not only the saltiness but also the wholel flavor is enhanced. |
| Example 32 | Device 2 | Commercial Fish Sausage | Cathodal Stimulation 0.5mA, Hold 1.0seconds thereafter Anodal Stimulation 0.3mA, Hold 1.0seconds | 4 | 2 | 1 | Not only the saltiness but also the whole flavor is enhanced. |
| Compa rison 7 | Device 4 | Commercial Fish Sausage | Anodal Stimulation 1.0mA, Hold 0.5seconds | 3 | 4 | 5 | Metaric tase is strong. |
| Comparison 8 | Device 4 | Commercial Fish Sausage | Cathodal Stimulation 1.0mA, Hold 0.5seconds | 2 | 4 | 1 | Irritating feeling exists when eating. |
| Example 33 | Device 2 | Commercial Steamed Chicken | Anodal Stimulation 1.0mA, Hold 0.5seconds | 4 | 3 | 2 | Sensed saltiness strongly when drinking. |
| Example 34 | Device 2 | Commercial Steamed Chicken | Anodal Stimulation 0.5mA, Hold 0.5seconds | 4 | 2 | 2 | Sensed saltiness strongly when drinking. |
| Example 35 | Device 2 | Commercial Steamed Chicken | Cathodal Stimulation 0.5mA, Hold 0.5seconds | 3 | 2 | 1 | Not only the saltiness but also the whole flavor is enhanced. |
| Example 36 | Device 2 | Commercial Steamed Chicken | Cathodal Stimulation 0.5mA, Hold 1.0seconds thereafter Anodal Stimulation 1.0mA, Hold 1.0seconds | 5 | 2 | 3 | Not only the saltiness but also the whole flavor is enhanced. |
| Example 37 | Device 2 | Commercial Steamed Chicken | Cathodal Stimulation 1.0mA, Hold 1.0seconds thereafter Anodal Stimulation 0.5mA, Hold 1.0seconds | 5 | 3 | 2 | Not only the saltiness but also the whole flavor is enhanced. |
| Example 38 | Device 2 | Commercial Steamed Chicken | Cathodal Stimulation 0.5mA, Hold 1.0seconds thereafter Anodal Stimulation 0.5mA, Hold 1.0seconds | 5 | 2 | 2 | Not only the saltiness but also the whole flavor is enhanced. |
| Example 39 | Device 2 | Commercial Steamed Chicken | Cathodal Stimulation 0.5mA, Hold 1.0seconds thereafter Anodal Stimulation 0.2mA, Hold 1.0seconds | 4 | 2 | 1 | Not only the saltiness but also the whole flavor is enhanced. |
| Comparison 9 | Device 4 | Commercial Steamed Chicken | Anodal Stimulation 0.5mA, Hold 0.5seconds | 3 | 4 | 4 | Metaric tase is strong. |
| Comparison 10 | Device 4 | Commercial Steamed Chicken | Cathodal Stimulation 0.5mA, Hold 0.5seconds | 2 | 4 | 1 | Irritating feeling exists when eating. |
| Example 40 | Device 2 | Commercial Cup Noodle (2xdilution) | Anodal Stimulation 0.5mA, Hold 0.5seconds | 4 | 2 | 2 | Sensed saltiness strongly when drinking. |
| Example 41 | Device 2 | Commercial Cup Noodle (2xdilution) | Cathodal Stimulation 0.5mA, Hold 0.5seconds | 3 | 2 | 1 | Not only the saltiness but also the whole flavor is enhanced. |
| Example 42 | Device 2 | Commercial Cup Noodle (2xdilution) | Cathodal Stimulation 0.5mA, Hold 0.5seconds thereafter Anodal Stimulation 0.5mA, Hold 0.3seconds | 5 | 2 | 1 | Not only the saltiness but also the whole flavor is enhanced. |
| Comparison 11 | Device 4 | Commercial Cup Noodle (2xdilution) | Anodal Stimulation 0.5mA, Hold 0.5seconds | 3 | 4 | 4 | Metaric tase is strong. |
| Comparison 12 | Device 4 | Commercial Cup Noodle (2xdilution) | Cathodal Stimulation 0.5mA, Hold 0.5seconds | 2 | 4 | 1 | Irritating feeling exists when eating. |

In Table 6, Examples 23 to 42 are evaluation results of cases where either the anodal stimulation or the cathodal stimulation was implemented alone according to the current waveform of FIG. 4, or the cathodal stimulation was implemented first and thereafter the anodal stimulation was implemented according to the current waveform of FIG. 5. Comparisons 5 to 12 are evaluation results of cases where, using either Device 3 or Device 4 from which the noise reduction parts were omitted, either the anodal stimulation or the cathodal stimulation was implemented alone, or the cathodal stimulation was implemented first and thereafter the anodal stimulation was implemented. The type of general food sample and the electric stimulation conditions in each example are as described in Table 6. In Table 6, representative comments of subjects are included. Incidentally, the increase time of the current value when implementing the anodal stimulation alone or when starting the cathodal stimulation first is 0.01 seconds, and the rate of increase is within the same range as in Examples 1 to 11 of Table 4. The reversal time in the cases where polarity reversal is implemented is 0.1 seconds or shorter as in the examples of Table 5. In the cases where an instant egg soup and an instant miso soup were adopted as the general food samples, to take the sample, subjects drank the sample by directly putting their mouth on a container holding the sample, without using a straw.

According to the results of Table 6, it can be confirmed that in Examples 23 to 42 in which noise reduction was implemented, the effect of mitigating the unpleasant sensations is improved as compared with that in Comparisons 5 to 12 in which noise reduction was omitted for the same samples. In particular, the effect of noise reduction appears prominently with respect to the irritating feeling and the metallic taste. For example, according to a comparison between Example 23 and Comparison 5, the evaluation of the irritating feeling is improved from "4" to "1", and the evaluation of the metallic taste is improved form "5" to "1". Further, in the cases where polarity reversal was implemented, a further improvement can be recognized compared with the cases where the anodal stimulation or the cathodal stimulation was implemented alone, regardless of the type of general food sample. For example, according to a comparison between Example 23 and Example 25 or a comparison between Example 29 and Example 31, the evaluation of the saltiness enhancing effect is improved from "4" to "5", despite the set value of the current value during the anodal stimulation being reduced from 1.0 mA to 0.5 mA. In comparisons between these Examples, the holding time of the anodal stimulation is different, and the holding time in the case where polarity reversal was implemented is set to be longer. However, when Example 40 and Example 42 are compared, it can be seen that the holding time of the anodal stimulation in Example 42 is 0.3 seconds and shorter than the holding time of 0.5 seconds in Example 40, and that, nevertheless, the evaluation on the saltiness enhancing effect in Example 42 is higher if the current value is the same. Thus, it is inferred that whether polarity reversal is implemented or not, rather than whether the holding time of the anodal stimulation is long or short, has a relatively greater influence on the effect of altering a taste. From the comments of the subjects, it can also be confirmed that the whole flavor was found to be enhanced in many of Examples.

### (5) Confirmation of Effects Due to Difference in Rate of Increase in Current Value

A test that was conducted to confirm an influence that a difference in the rate of increase in the current value during current supply had on the saltiness enhancing effect etc. will be described with reference to Table 7. Table 7 shows, as Examples 43 and 44, results of evaluating the saltiness enhancing effect etc. using Device 2 with a commercial fish sausage adopted as the general food sample. The reason why a commercial fish sausage was selected as the sample is that it was deemed to be suitable for confirming the mitigating effect on the irritating feeling, because a food contained less moisture than a drink and the irritating feeling during ingestion tended to appear more noticeably in a food.

**Table 7**

| | Device Used | Sample | Electric Stimulation Condition | Saltiness | Irritating Feeling | Metaric Taste | Comment |
|---|---|---|---|---|---|---|---|
| Example 43 | Device 2 | Commercial Fish Sausage | Anodal Stimulation, 1.0mA over 0.1seconds, Hold 0.5seconds | 4 | 2 | 2 | Sensed strong saltiness. |
| Example 44 | Device 2 | Commercial Fish Sausage | Cathodal Stimulation, 1.0mA over 0.1seconds, Hold 0.5seconds | 3 | 2 | 1 | Not only the saltiness but also the wholel flavor is enhanced. |

In Example 43 of Table 7, only the anodal stimulation is implemented, and the electric stimulation conditions match those of Example 29 having the same sample as the target, except that the increase time until the current value reaches the set value of 1.0 mA is changed to 0.1 seconds (which is 0.01 seconds in Example 29). In Example 44, only the cathodal stimulation is implemented, and the electric stimulation conditions match those of Example 30 having the same sample as the target, except that the increase time until the current value reaches the set value of 1.0 mA is changed to 0.1 seconds (which is 0.01 seconds in Example 30). As is clear from a comparison between Example 29 and Example 43 and a comparison between Example 30 and Example 44, it can be confirmed that the irritating feeling is further weakened in Examples 43 and 44 in which the increase time was set to be relatively long.

Various aspects of the present invention that are derived from each of the above-described embodiment, modifications, and Examples will be described below. Incidentally, in the following description, corresponding constituent elements shown in the accompanying drawings are added in parentheses to facilitate understanding of the aspects of the present invention, but this does not mean that the present invention is thereby limited to the form shown in the drawings.

A taste presentation device (10) according to one aspect of the present invention is a device which comprises a first electrode (11) and a second electrode (12) that are provided to allow an electric circuit to be formed between a food or drink (2) to be taken by a user (1) and a body of the user, and an electric stimulus generation unit (13) that supplies a current for generating an electric stimulation between the first electrode and the second electrode, wherein the electric stimulus generation unit includes a noise reduction unit (17A, 17B) that reduces a noise component in the current.

According to studies by the inventors, it has been revealed that, when attempting to alter a taste by using electric stimulation, a noise component included in a current causes exacerbation of an irritating feeling and a metallic taste, with these unpleasant sensations tending to intensify as the noise component becomes larger. Therefore, by reducing the noise component in the current supplied between the electrodes by the noise reduction unit, it is possible to mitigate the influence that the noise component has on the irritating feeling and the metallic taste and thereby enhance the intended taste alteration effect.

In the above-described aspect, after starting to supply the current, the electric stimulus generation unit may change a direction of the current such that a polarity of the first electrode and the second electrode is reversed. When the polarity is reversed in the middle of generating the electric stimulation by supplying the current, it is possible to restrain the absolute value of a current value to a relatively low level and yet to increase the amount of change in the current value during polarity reversal to effectively draw the taste alteration effect.

The electric stimulus generation unit may start to supply the current in a state of cathodal stimulation in which the second electrode serves as a cathode, and thereafter change the direction of the current such that the state switches to a state of anodal stimulation in which the second electrode serves as an anode. In the cathodal stimulation, the unpleasant sensations of the user can be mitigated to a comparatively low degree while the taste alteration effect is weak, whereas in the anodal stimulation, the taste alteration effect is comparatively high while the unpleasant sensations, especially the metallic taste tends to be induced comparatively intensely. Therefore, by implementing the cathodal stimulation first to mitigate the unpleasant sensations and thereafter implementing the anodal stimulation by reversing the polarity, it is possible to secure a large amount of change in the current value during the reversal to thereby enhance the taste alteration effect, and at the same time to restrain a set value of the current during the anodal stimulation to a relatively low level to thereby restrain the unpleasant sensations from increasing.

The electric stimulus generation unit may change the direction of the current such that the cathodal stimulation in which a predetermined current set value (Is1) is held is implemented for a holding time (Ty1) of 0.1 seconds or longer, and that the anodal stimulation is implemented after the holding time. Setting the holding time of the cathodal stimulation to the aforementioned range can enhance the effect when implementing the cathodal stimulation first.

The electric stimulus generation unit may change the direction of the current such that a reversal of the polarity is completed within a reversal time (Tx2) of 0.5 seconds or shorter. Thus, the effect of implementing the polarity reversal can be effectively drawn.

The electric stimulus generation unit may, when starting to supply the current, gradually increase a current value in such a manner that a rate of change in the current value is restricted to 0.1 mA/ms or lower. By increasing the current value while restricting the rate of change in the current value within the aforementioned range, it is possible to mitigate exacerbation of the irritating feeling accompanying a rapid increase in the current value and reliably draw the alteration effect, such as taste enhancement.

The first electrode may be provided in contact with the body of the user, and the second electrode may be provided in contact with the food or drink. When the electrodes are thus disposed, an electric circuit can be formed between the food or drink and the body of the user during intake of the food or drink.

The noise reduction unit (17A) may be provided to reduce a noise component originating from a power source. When the electric stimulus generation unit includes a booster circuit (14), the noise reduction unit (17B) may be provided to reduce a noise component corresponding to an internal oscillation frequency in the booster circuit. According to these forms, the noise components that exacerbate the irritating feeling and the metallic taste can be effectively reduced.

The present invention may be configured as a taste presentation method including a step of providing a first electrode (11) and a second electrode (12) to allow an electric circuit to be formed between a food or drink (2) to be taken by a user (1) and a body of the user, and a step of supplying a current for generating an electric stimulation between the first electrode and the second electrode, wherein, in the step of supplying the current, a noise component in the current is reduced.

### REFERENCE SIGNS LIST

10 taste presentation device
11 first electrode
12 second electrode
13 electric stimulus generation part (electric stimulus generation unit)
14 booster circuit
17A,17B noise reduction part (noise reduction unit)
Further embodiments are defined as E1 to E10, as follows:
E1. A taste presentation device comprising:
   a first electrode and a second electrode that are provided to allow an electric circuit to be formed between a food or drink to be taken by a user and a body of the user; and
   an electric stimulus generation unit that supplies a current for generating an electric stimulation between the first electrode and the second electrode,
   wherein the electric stimulus generation unit includes a noise reduction unit that reduces a noise component in the current.
E2. The taste presentation device according to embodiment 1, wherein, after starting to supply the current, the electric stimulus generation unit changes a direction of the current such that a polarity of the first electrode and the second electrode is reversed.
E3. The taste presentation device according to embodiment 2, wherein the electric stimulus generation unit starts to supply the current in a state of a cathodal stimulation in which the second electrode serves as a cathode, and thereafter changes the direction of the current such that the state switches to a state of an anodal stimulation in which the second electrode serves as an anode.
E4. The taste presentation device according to embodiment 3, wherein the electric stimulus generation unit changes the direction of the current such that the cathodal stimulation in which a predetermined current set value is held is implemented for a holding time of 0.1 seconds or longer, and that the anodal stimulation is implemented after the holding time.
E5. The taste presentation device according to any one of embodiments 2 to 4, wherein the electric stimulus generation unit changes the direction of the current such that a reversal of the polarity is completed within a reversal time of 0.5 seconds or shorter.
E6. The taste presentation device according to any one of embodiments 1 to 5, wherein, when starting to supply the current, the electric stimulus generation unit gradually increases a current value in such a manner that a rate of change in the current value is restricted to 0.1 mA/ms or lower.
E7. The taste presentation device according to any one of embodiments 1 to 6, wherein the first electrode is provided in contact with the body of the user, and the second electrode is provided in contact with the food or drink.
E8. The taste presentation device according to any one of embodiments 1 to 7, wherein the noise reduction unit is provided to reduce a noise component originating from a power source.
E9. The taste presentation device according to any one of embodiments 1 to 8, wherein the electric stimulus generation unit includes a booster circuit, and the noise reduction unit is provided to reduce a noise component corresponding to an internal oscillation frequency in the booster circuit.
E10. A taste presentation method including:
   a step of providing a first electrode and a second electrode to allow an electric circuit to be formed between a food or drink to be taken by a user and a body of the user, and
   a step of supplying a current for generating an electric stimulation between the first electrode and the second electrode,
   wherein, in the step of supplying the current, a noise component in the current is reduced.

## Claims

1. A taste presentation device comprising:
a first electrode and a second electrode that are provided to allow an electric circuit to be formed between a food or drink to be taken by a user and a body of the user; and
an electric stimulus generation unit that supplies a current for generating an electric stimulation between the first electrode and the second electrode,
wherein the electric stimulus generation unit includes a noise reduction unit that reduces a noise component in the current.

2. The taste presentation device according to claim 1, wherein, after starting to supply the current, the electric stimulus generation unit changes a direction of the current such that a polarity of the first electrode and the second electrode is reversed.

3. The taste presentation device according to claim 2, wherein the electric stimulus generation unit starts to supply the current in a state of a cathodal stimulation in which the second electrode serves as a cathode, and thereafter changes the direction of the current such that the state switches to a state of an anodal stimulation in which the second electrode serves as an anode.

4. The taste presentation device according to claim 3, wherein the electric stimulus generation unit changes the direction of the current such that the cathodal stimulation in which a predetermined current set value is held is implemented for a holding time of 0.1 seconds or longer, and that the anodal stimulation is implemented after the holding time.

5. The taste presentation device according to any one of claims 2 to 4, wherein the electric stimulus generation unit changes the direction of the current such that a reversal of the polarity is completed within a reversal time of 0.5 seconds or shorter.

6. The taste presentation device according to any one of claims 1 to 5, wherein the first electrode is provided in contact with the body of the user, and the second electrode is provided in contact with the food or drink.

7. The taste presentation device according to any one of claims 1 to 6, wherein the noise reduction unit is provided to reduce a noise component originating from a power source.

8. The taste presentation device according to any one of claims 1 to 7, wherein the electric stimulus generation unit includes a booster circuit, and the noise reduction unit is provided to reduce a noise component corresponding to an internal oscillation frequency in the booster circuit.
